# EUROPÄISCHE PATENTANMELDUNG

(11) **EP 1 428 519 A2**
(43) Veröffentlichungstag der Anmeldung: **16.06.2004**
(21) Anmeldenummer: 03027424.5
(22) Anmeldetag: 28.11.2003
(51) Int. Cl.: A61K 7/32

(54) **5-Lipoxigenase-inhibitoren in Deodorantien und Antitranspirantien**

(30) Priorität: 10.12.2002 DE 10257738
(71) Anmelder: Henkel Kommanditgesellschaft auf Aktien, 40589 Düsseldorf (DE)
(72) Erfinder: Banowski, Bernhard, 40597 Düsseldorf (DE); Waldmann-Laue, Marianne, Dr., 40789 Monheim (DE); Wadle, Armin, Dr., 40699 Erkrath (DE); Siegert, Petra, Dr., 42781 Haan (DE); Sättler, Andrea, Dr., 40255 Düsseldorf (DE)

(57) **Zusammenfassung**

Gegenstand der Erfindung ist die Verwendung von ausgewählten 5-Lipoxigenaseinhibierenden Substanzen in einer kosmetischen Deodorant- oder Antitranspirant-Zusammensetzung zur Verringerung des durch die Bildung von gesättigten und ungesättigten C₆-C₁₂-Aldehyden aus den ungesättigten Fettsäuren der Haut und der Kopfhaut verursachten Körpergeruchs.

## Beschreibung

Gegenstand der Erfindung ist die Verwendung von ausgewählten 5-Lipoxigenase-inhibierenden Substanzen in einer kosmetischen Deodorant- oder Antitranspirant-Zusammensetzung zur Verringerung des durch die Umwandlung von natürlichen Fetten der Haut und der Kopfhaut in gesättigte und ungesättigte Aldehyde verursachten Körpergeruchs.

Apokriner Schweiß stellt eine komplexe Mischung dar, die unter anderem Sebum und andere Fette, Steroide, Cholesterin sowie ca. 10 % Eiweiße enthält.
Axillarer Körpergeruch ist im wesentlichen auf flüchtige kurz- und mittelkettige Fettsäuren zurückzuführen, die aus bei der Spaltung von Hautlipiden freigesetzten, verzweigten langkettigen Fettsäuren gebildet werden. Aus den langkettigen verzweigten Fettsäuren werden durch die hydrolytischen Enzyme von Corynebacterium A sowohl kurzkettige C₂ - C₅-Fettsäuren als auch mittelkettige C₆ - C₁₂-Fettsäuren gebildet, die hauptsächlich für den axillaren Körpergeruch verantwortlich sind.
Eine weitere Verbindungsklasse, die ebenfalls bei der bakteriellen Zersetzung der Schweiß-Bestandteile entsteht und zum Körpergeruch beiträgt, sind gesättigte und ungesättigte Aldehyde, vor allem solche mit einer Kettenlänge von C₆ - C₁₂, insbesondere Hexanal, Heptanal, Octenal und Nonenal. Diese entstehen durch β-Spaltung aus den Hydroperoxiden, die unter Einwirkung der 5-Lipoxigenase auf ungesättigte Fettsäuren gebildet werden.

Die erfindungsgemäß wirksamen Deodorant-Zusammensetzungen können an dieser Stelle eingreifen und die Tätigkeit der bakteriellen 5-Lipoxigenase hemmen. Damit unterscheiden sie sich von den rein bakteriostatischen oder bakteriziden Zusammensetzungen des Standes der Technik, die den Nachteil aufweisen können, die natürliche Mikroflora der Haut zu beeinträchtigen.

Die Bekämpfung von Körpergeruch durch die Hemmung von 5-Lipoxigenase ist im Stand der Technik bekannt aus den Druckschriften EP 955 035 A1, WO 97/07780 A1 und DD 293 958 A1.

Aufgabe der vorliegenden Erfindung war es, weitere 5-Lipoxigenase-inhibierende Wirkstoffe zu identifizieren, um eine größere Variabilität, Flexibilität und Hautverträglichkeit bei der Formulierung kosmetischer Deodorantien zu ermöglichen. Die Identifizierung bekannter kosmetischer Wirkstoffe als 5-Lipoxigenase-Inhibitoren ermöglicht darüber hinaus, die Dosierung dieser Wirkstoffe herabzusetzen. Die enzyminhibierende Wirkung zeigt sich häufig bereits bei niedrigen Wirkstoffkonzentrationen, bei denen noch keine bakteriostatische oder bakterizide Wirkung gefunden wird.

Gegenstand der vorliegenden Erfindung ist die Verwendung von mindestens einer 5-Lipoxigenase-inhibierenden Substanz, ausgewählt aus :
Aminodicarbonsäuren mit einer C-Kettenlänge von 3 - 6 Kohlenstoffatomen sowie deren physiologisch verträglichen Salzen,
N-alkylierten C₂-C₁₁-Aminosäuren mit einem C₁-C₂₂-Alkylrest sowie deren physiologisch verträglichen Salzen,
N-acylierten C₂-C₁₁-Aminosäuren mit einem C₂-C₂₂-Acylrest sowie deren physiologisch verträglichen Salzen,
Hefeextrakten,
α-Bisabolol,
α-Liponsäure und
den physiologisch verträglichen Salzen von Sterolsulfaten,
in einer kosmetischen Deodorant- oder Antitranspirant-Zusammensetzung zur Verringerung des durch die Bildung von gesättigten und ungesättigten C₆-C₁₂-Aldehyden aus den ungesättigten Fettsäuren der Haut und der Kopfhaut verursachten Körpergeruchs.

In einer bevorzugten Ausführungsform sind die erfindungsgemäß verwendeten Aminodicarbonsäuren mit einer C-Kettenlänge von 3 - 6 Kohlenstoffatomen ausgewählt aus Aminomalonsäure, Aminobernsteinsäure (= Asparaginsäure), Aminoglutarsäure und Aminoadipinsäure sowie deren physiologisch verträglichen Salzen. Besonders bevorzugt sind Asparaginsäure und ihre physiologisch verträglichen Salze, insbesondere Kaliumaspartat und Magnesiumaspartat.
Erfindungsgemäß werden die Aminodicarbonsäuren mit einer C-Kettenlänge von 3 - 6 Kohlenstoffatomen sowie deren Salze in Mengen von 0,01 bis 5 Gew.-%, bevorzugt 0,1 bis 2 Gew.-% und besonders bevorzugt 0,5 bis 1 Gew.-%, jeweils bezogen auf die gesamte kosmetische Zusammensetzung, eingesetzt.

In einer weiteren bevorzugten Ausführungsform sind die erfindungsgemäß verwendeten N-alkylierten C₂-C₁₁-Aminosäuren mit einem C₁-C₂₂-Alkylrest ausgewählt aus Alanin, Glutaminsäure, Pyroglutaminsäure, Lysin, Arginin, Histidin, Valin, Leucin, Isoleucin, Prolin, Tryptophan, Phenylalanin, Methionin, Glycin, Serin, Tyrosin, Threonin, Cystein, Asparagin und Glutamin sowie deren physiologisch verträglichen Salzen, die am Stickstoffatom der Aminogruppe einen C₁-C₂₂-Alkylrest, ausgewählt aus einer Gruppe Methyl, Ethyl, Propyl, Butyl, Pentyl, Hexyl, Heptyl, Octyl, Nonyl, Decyl, Undecyl, Dodecyl (Lauryl), Tridecyl, Tetradecyl (Myristyl), Pentadecyl, Hexadecyl (Palmityl, Cetyl), Heptadecyl, Octadecyl (Stearyl), Nonadecyl, Eicosanyl (Arachidyl) und Behenyl, aufweisen. Besonders bevorzugt ist N-Methylglycin (= Sarcosin).
Erfindungsgemäß werden die N-alkylierten C₂-C₁₁-Aminosäuren mit einem C₁-C₂₂-Alkylrest sowie deren physiologisch verträglichen Salze in Mengen von 0,01 bis 10 Gew.-%, bevorzugt 0,1 bis 5 Gew.-% und besonders bevorzugt 0,5 bis 2 Gew.-%, jeweils bezogen auf die gesamte kosmetische Zusammensetzung, eingesetzt.

In einer weiteren bevorzugten Ausführungsform sind die erfindungsgemäß verwendeten N-acylierten C₂-C₁₁-Aminosäuren mit einem C₂-C₂₂-Acylrest ausgewählt aus Glutaminsäure, Pyroglutaminsäure, Lysin, Arginin, Histidin, Valin, Leucin, Isoleucin, Prolin, Tryptophan, Phenylalanin, Methionin, Glycin, Serin, Tyrosin, Threonin, Cystein, Asparagin und Glutamin, die mit einem C₂ - C₂₂-Acylrest an der Aminogruppe derivatisiert sind, sowie deren physiologisch verträglichen Salzen. Die Aminosäuren können einzeln oder im Gemisch eingesetzt werden. Erfindungsgemäß geeignet sind insbesondere Aminosäuren-Gemische, die aus Pflanzen, insbesondere Getreidepflanzen, gewonnen wurden. Der C₂ - C₂₂-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Besonders bevorzugt sind Natriumcocoylaminosäuren, Natriumoctanoylglutamat, Natriumdecanoylglutamat, Natriumlauroylglutamat, Natriummyristoylglutamat, Natriumcetoylglutamat und Natriumstearoylglutamat und die Lauroyl-Derivate von aus Getreidepflanzen gewonnenen Aminosäuren.

Die Getreidepflanzen, aus denen die erfindungsgemäß geeigneten Aminosäuren gewonnen werden, unterliegen keiner Einschränkung. Geeignet sind beispielsweise Hafer, Weizen, Gerste und Roggen; besonders geeignet ist Hafer.
Ein besonders bevorzugt geeigneter 5-Lipoxigenase-Inhibitor ist das Handelsprodukt Seppicalm von der Firma Seppic mit der INCI-Bezeichnung "Sodium Cocoyl Aminoacids, Sarcosine, Potassium Aspartate, Magnesium Aspartate".
Erfindungsgemäß werden die N-acylierten C₂-C₁₁-Aminosäuren mit einem C₂-C₂₂-Acylrest sowie deren physiologisch verträglichen Salze in Mengen von 0,01 bis 10 Gew.-%, bevorzugt 0,1 bis 5 Gew.-% und besonders bevorzugt 0,5 bis 2 Gew.-%, jeweils bezogen auf die gesamte kosmetische Zusammensetzung, eingesetzt.

In einer weiteren bevorzugten Ausführungsform werden die erfindungsgemäß als 5-Lipoxigenase-Inhibitoren verwendeten Hefeextrakte in Mengen von 0,001 bis 5 Gew.-%, bevorzugt 0,01 bis' 2 Gew.-% und besonders bevorzugt 0,1 bis 1 Gew.-%, jeweils bezogen auf die gesamte kosmetische Zusammensetzung, eingesetzt. Ein besonders bevorzugt verwendetes Handelsprodukt ist Drieline (INCI-Bezeichnung "Sorbitol, Yeast Extract"), erhältlich von der Firma Lanatech.

In einer weiteren bevorzugten Ausführungsform wird der erfindungsgemäß verwendete 5-Lipoxigenase-Inhibitor α-Bisabolol in Mengen von 0,001 bis 5 Gew.-%, bevorzugt 0,01 bis 2 Gew.-% und besonders bevorzugt 0,1 bis 1 Gew.-%, jeweils bezogen auf die gesamte kosmetische Zusammensetzung, eingesetzt.

In einer weiteren bevorzugten Ausführungsform wird der erfindungsgemäß verwendete 5-Lipoxigenase-Inhibitor α-Liponsäure in Mengen von 0,001 bis 5 Gew.-%, bevorzugt 0,01 bis 2 Gew.-% und besonders bevorzugt 0,1 bis 1 Gew.-%, jeweils bezogen auf die gesamte kosmetische Zusammensetzung, eingesetzt.

In einer weiteren bevorzugten Ausführungsform sind die erfindungsgemäß als 5-Lipoxigenase-Inhibitoren verwendeten physiologisch verträglichen Salze der Sterolsulfate ausgewählt aus den Salzen von β-Sitosterolsulfat, Ergosterolsulfat, Stigmasterolsulfat, Cholesterolsulfat und Lanosterolsulfat. Besonders bevorzugt sind die Salze von β-Sitosterolsulfat. Die Sterolsulfatsalze werden in Mengen von 0,001 bis 5 Gew.-%, bevorzugt 0,01 bis 2 Gew.-% und besonders bevorzugt 0,1 bis 1 Gew.-%, jeweils bezogen auf die gesamte kosmetische Zusammensetzung, eingesetzt. Die Sterolsulfatsalze können dabei sowohl einzeln als auch in beliebigen Mischungen eingesetzt werden. Ein besonders bevorzugt eingesetztes Handelsprodukt ist Phytocohesine (INCI-Bezeichnung "Sodium Beta-Sitosteryl Sulfate"), erhältlich von der Firma Vincience.

Die physiologisch verträglichen Salze der vorgenannten 5-Lipoxigenase-Inhibitoren sind ausgewählt aus den Ammonium-, Alkalimetall-, Magnesium-, Calcium-, Aluminium-, Zinkund Mangan-Salzen. Bevorzugt sind die Natrium-, Kalium-, Magnesium-, Aluminium-, Zink- und Mangan-Salze.

Ein weiterer Gegenstand der vorliegenden Erfindung ist ein Verfahren zur Verringerung von Körpergeruch mittels Inhibierung von 5-Lipoxigenase auf der Haut, das dadurch gekennzeichnet ist, dass eine kosmetische Deodorant- oder Antitranspirant-Zusammensetzung, enthaltend mindestens eine 5-Lipoxigenase-inhibierende Substanz, ausgewählt aus Aminodicarbonsäuren mit einer C-Kettenlänge von 3 - 6 Kohlenstoffatomen sowie deren physiologisch verträglichen Salzen,
N-alkylierten C₂-C₁₁-Aminosäuren mit C₁-C₂₂-Alkylresten sowie deren physiologisch verträglichen Salzen,
N-acylierten C₂-C₁₁-Aminosäuren mit C₂-C₂₂-Acylresten sowie deren physiologisch verträglichen Salzen,
Hefeextrakten,
α-Bisabolol,
α-Liponsäure und
den physiologisch verträglichen Salzen von Sterolsulfaten,
auf die Haut, insbesondere auf die Haut der Achselhöhlen, aufgetragen wird.

Ein weiterer Gegenstand der vorliegenden Erfindung ist die Verwendung von α-Liponsäure gegen Hautirritationen, zur Entzündungshemmung und zur Hautberuhigung.

Die kosmetischen Deodorant- oder Antitranspirant-Zusammensetzungen, die die erfindungsgemäß verwendeten 5-Lipoxigenase-Inhibitoren enthalten, können als Puder, in Stiftform, als Aerosolspray, Pumpspray, flüssige und gelförmige Roll on-Applikation, Creme, Gel und als getränktes flexibles Substrat vorliegen.
Deodorant- oder Antitranspirant-Stifte können in gelierter Form, auf wasserfreier Wachsbasis und auf Basis von W/O-Emulsionen und O/W-Emulsionen vorliegen. Gelstifte können auf der Basis von Fettsäureseifen, Dibenzylidensorbitol, N-Acylaminosäureamiden, 12-Hydroxystearinsäure und anderen Gelbildnern hergestellt werden.

Aerosolsprays, Pumpsprays, Roll on-Applikationen und Cremes können als Wasser-in-Öl-Emulsion, Öl-in-Wasser-Emulsion, Siliconöl-in-Wasser-Emulsion, Wasser-in-Öl-Mikroemulsion, Öl-in-Wasser-Mikroemulsion, Siliconöl-in-Wasser-Mikroemulsion, wasserfreie Suspension, alkoholische und hydroalkoholische Lösung, wässriges Gel und als Öl vorliegen. Alle genannten Zusammensetzungen können verdickt sein, beispielsweise auf der Basis von Fettsäureseifen, Dibenzylidensorbitol, N-Acylaminosäureamiden, 12-Hydroxystearinsäure, Polyacrylaten vom Carbomer- und Carbopol-Typ, Polyacrylamiden und Polysacchariden, die chemisch und/oder physikalisch modifiziert sein können.
Die Emulsionen und Mikroemulsionen können transparent, translucent oder opak sein.

Die kosmetischen Deodorant- oder Antitranspirant-Zusammensetzungen, die die erfindungsgemäß verwendeten 5-Lipoxigenase-Inhibitoren enthalten, können weiterhin Fettstoffe enthalten. Unter Fettstoffen sind Fettsäuren, Fettalkohole, natürliche und synthetische kosmetische Ölkomponenten sowie natürliche und synthetische Wachse zu verstehen, die sowohl in fester Form als auch flüssig in wässriger oder öliger Dispersion vorliegen können.
Als Fettsäuren können eingesetzt werden lineare und/oder verzweigte, gesättigte und/oder ungesättigte C₈₋₃₀-Fettsäuren. Bevorzugt sind C₁₀₋₂₂-Fettsäuren. Beispiele sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Besonders bevorzugt ist der Einsatz von Stearinsäure. Die eingesetzten Fettsäuren können eine oder mehrere Hydroxygruppen tragen. Bevorzugte Beispiele hierfür sind die α-Hydroxy-C₈-C₁8-Carbonsäuren sowie 12-Hydroxystearinsäure.
Die Einsatzmenge beträgt dabei 0,1 - 15 Gew.-%, bevorzugt 0,5 - 10 Gew.-%, besonders bevorzugt 1 - 5 Gew.-%, jeweils bezogen auf die gesamte Zusammensetzung.
Als Fettalkohole können eingesetzt werden gesättigte, ein- oder mehrfach ungesättigte, verzweigte oder unverzweigte Fettalkohole mit 6 - 30, bevorzugt 10 - 22 und ganz besonders bevorzugt 12 - 22 Kohlenstoffatomen. Einsetzbar im Sinne der Erfindung sind z.B. Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole.
Für Stiftformulierungen werden häufig Wachse verwendet. Als natürliche oder synthetische Wachse können erfindungsgemäß eingesetzt werden feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie z. B. Bienenwachse und andere Insektenwachse, Walrat, Schellackwachs, Wollwachs und Bürzelfett, weiterhin Mineralwachse, wie z. B. Ceresin und Ozokerit oder die petrochemischen Wachse, wie z. B. Petrolatum, Paraffinwachse, Microwachse aus Polyethylen oder Polypropylen und Polyethylenglycolwachse. Es kann vorteilhaft sein, hydrierte oder gehärtete Wachse einzusetzen. Weiterhin sind auch chemisch modifizierte Wachse, insbesondere die Hartwachse, z. B. Montanesterwachse, Sasolwachse und hydrierte Jojobawachse, einsetzbar.
Weiterhin geeignet sind die Triglyceride gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs®) oder Polyolen mit 2 - 6 C-Atomen, Fettsäuremonoalkanolamide mit einem C₁₂₋₂₂-Acylrest und einem C₂₋₄-Alkanolrest, Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, darunter z. B. synthetische Fettsäure-Fettalkoholester wie Stearylstearat oder Cetylpalmitat, Ester aus aromatischen Carbonsäuren, Dicarbonsäuren bzw. Hydroxycarbonsäuren (z. B. 12-Hydroxystearinsäure) und gesättigten und/oder ungesättigten, verzweigten und/ oder unverzweigten Alkoholen einer Kettenlänge von 1 bis 80 C-Atomen, Lactide langkettiger Hydroxycarbonsäuren und Vollester aus Fettalkoholen und Di- und Tricarbonsäuren, z. B. Dicetylsuccinat oder Dicetyl-/stearyladipat, sowie Mischungen dieser Substanzen, sofern die einzelnen Wachskomponenten oder ihre Mischung bei Raumtemperatur fest sind.
Besonders bevorzugt ist, die Wachskomponenten zu wählen aus der Gruppe der Ester aus gesättigten, unverzweigten Alkancarbonsäuren einer Kettenlänge von 14 bis 44 C-Atomen und gesättigten, unverzweigten Alkoholen einer Kettenlänge von 14 bis 44 C-Atomen, sofern die Wachskomponente oder die Gesamtheit der Wachskomponenten bei Raumtemperatur fest sind. Insbesondere vorteilhaft können die Wachskomponenten aus der Gruppe der C₁₆₋₃₆-Alkylstearate, der C₁₀₋₄₀-Alkylstearate, der C₂₋₄₀-Alkylisostearate, der C₂₀₋₄₀-Dialkylester von Dimersäuren, der C₁₈₋₃₈-Alkylhydroxystearoylstearate, der C₂₀₋₄₀-Alkylerucate gewählt werden, ferner sind C₃₀₋₅₀-Alkylbienenwachs sowie Cetearylbehenat einsetzbar. Auch Silikonwachse, zum Beispiel Stearyltrimethylsilan/Stearylalkohol sind gegebenenfalls vorteilhaft. Besonders bevorzugte Wachskomponenten sind die Ester aus gesättigten, einwertigen C₂₀-C₆₀-Alkoholen und gesättigten C₈-C₃₀-Monocarbonsäuren, insbesondere ein C₂₀-C₄₀-Alkylstearat bevorzugt, das unter dem Namen Kesterwachs® K82H von der Firma Koster Keunen Inc. erhältlich ist. Das Wachs oder die Wachskomponenten sollten bei 25° C fest sein, jedoch im Bereich von 35 - 95°C schmelzen, wobei ein Bereich von 45 - 85 °C bevorzugt ist. Natürliche, chemisch modifizierte und synthetische Wachse können alleine oder in Kombination eingesetzt werden.
Die Wachskomponenten sind in einer Menge von 0,1 bis 40 Gew.-%, bezogen auf die Gesamtzusammensetzung, vorzugsweise 1 bis 30 Gew.-% und insbesondere 5 - 15 Gew.-% enthalten.
Die erfindungsgemäßen Zusammensetzungen können weiterhin wenigstens ein unpolares oder polares flüssiges Öl, das natürlich oder synthetisch sein kann, enthalten. Die polare Ölkomponente kann ausgewählt sein aus pflanzlichen Ölen, z. B. Sonnenblumenöl, Olivenöl, Sojaöl, Rapsöl, Mandelöl, Jojobaöl und den flüssigen Anteilen des Kokosöls sowie synthetischen Triglyceridölen, aus Esterölen, das heißt den Estern von C₆-₃₀-Fettsäuren mit C₂₋₃₀―Fettalkoholen, aus Dicarbonsäureestern wie Di-n-butyladipat, Di-(2-ethylhexyl)-adipat und Di-(2-ethylhexyl)-succinat sowie Diolestern wie Ethylenglykoldioleat und Propylenglykoldi(2-ethylhexanoat), aus symmetrischen, unsymmetrischen oder cyclischen Estern der Kohlensäure mit Fettalkoholen, beispielsweise beschrieben in der DE-OS 197 56 454, Glycerincarbonat oder Dicaprylylcarbonat (Cetiol® CC), aus Mono,- Di- und Trifettsäureestern von gesättigten und/oder ungesättigten linearen und/oder verzweigten Fettsäuren mit Glycerin, z. B. Cutina® MD, aus verzweigten Alkanolen, z. B. Guerbet-Alkoholen mit einer einzigen Verzweigung am Kohlenstoffatom 2 wie 2-Hexyldecanol, 2-Octyldodecanol, Isotridecanol und Isohexadecanol, aus Alkandiolen, z. B. den aus Epoxyalkanen mit 12 - 24 C-Atomen durch Ringöffnung mit Wasser erhältlichen vicinalen Diolen, aus Etheralkoholen, z. B. den Monoalkylethern des Glycerins, des Ethylenglycols, des 1,2-Propylenglycols oder des 1,2-Butandiols, aus Dialkylethern mit jeweils 12 - 24 C-Atomen, z. B. den Alkyl-methylethern oder Di-n-alkylethern mit jeweils insgesamt 12 - 24 C-Atomen, insbesondere Di-n-octylether (Cetiol®OE ex Cognis), sowie aus Anlagerungsprodukten von Ethylenoxid und/oder Propylenoxid an ein- oder mehrwertige C₃₋₂₀-Alkanole wie Butanol und Glycerin, z. B. PPG-3-Myristylether (Witconol® APM), PPG-14-Butylether (Ucon Fluid® AP), PPG-15-Stearylether (Arlamol® E), PPG-9-Butylether (Breox® B25) und PPG-10-Butandiol (Macol® 57).
Die unpolare Ölkomponente kann ausgewählt sein aus flüssigen Paraffinölen, Isoparaffinölen, z. B. Isohexadecan und Isoeicosan, aus synthetischen Kohlenwasserstoffen, z. B. 1,3-Di-(2-ethyl-hexyl)-cyclohexan (Cetiol® S), sowie aus flüchtigen und nichtflüchtigen Siliconölen, die cyclisch, wie z. B. Decamethylcyclopentasiloxan und Dodecamethylcyclohexasiloxan, oder linear sein können, z. B. lineares Dimethylpolysiloxan, im Handel erhältlich z. B. unter der Bezeichnung Dow Corning® 190, 200, 244, 245, 344 oder 345 und Baysilon® 350 M.

Die erfindungsgemäßen Zusammensetzungen können weiterhin wenigstens einen wasserlöslichen Alkohol enthalten. Unter Wasserlöslichkeit versteht man erfindungsgemäß, dass sich wenigstens 5 Gew.-% des Alkohols bei 20 °C klar lösen oder aber ― im Falle langkettiger oder polymerer Alkohole - durch Erwärmen der Lösung auf 50 °C bis 60 °C in Lösung gebracht werden können. Geeignet sind je nach Darreichungsform einwertige Alkohole wie z. B. Ethanol, Propanol oder Isopropanol. Weiterhin geeignet sind wasserlösliche Polyole. Hierzu zählen wasserlösliche Diole, Triole und höherwertige Alkohole sowie Polyethylenglycole. Unter den Diolen eignen sich C₂-C₁₂-Diole, insbesondere 1,2-Propylenglycol, Dipropylenglycol, Tripropylenglycol, Butylenglycole wie z. B. 1,2-Butylenglycol, 1,3-Butylenglycol und 1,4-Butylenglycol, Hexandiole wie z. B. 1,6-Hexandiol. Weiterhin bevorzugt geeignet sind Glycerin und insbesondere Diglycerin und Triglycerin, 1,2,6-Hexantriol sowie die Polyethylenglycole (PEG) PEG-400, PEG-600, PEG-1000, PEG-1550, PEG-3000 und PEG-4000.
Die Menge des Alkohols oder des Alkohol-Gemisches in den erfindungsgemäßen Zusammensetzungen beträgt 1 - 50 Gew.-% und vorzugsweise 5 - 40 Gew.-%, bezogen auf die gesamte Zusammensetzung. Erfindungsgemäß kann sowohl ein Alkohol als auch ein Gemisch mehrerer Alkohole eingesetzt werden.

Die erfindungsgemäßen Zusammensetzungen können im wesentlichen wasserfrei sein, das heißt maximal 5 Gew.-%, bevorzugt maximal 1 Gew.-% Wasser enthalten. In wasserhaltigen Darreichungsformen beträgt der Wassergehalt 5 - 98 Gew.-%, bevorzugt 10 - 90 und besonders bevorzugt 15 - 85 Gew.-%, bezogen auf die gesamte Zusammensetzung.

Die erfindungsgemäßen Zusammensetzungen können weiterhin wenigstens ein hydrophil modifiziertes Silicon enthalten. Sie ermöglichen die Formulierung hochtransparenter Zusammensetzungen, reduzieren die Klebrigkeit und hinterlassen ein frisches Hautgefühl. Unter hydrophil modifizierten Siliconen werden erfindungsgemäß Polyorganosiloxane mit hydrophilen Substituenten verstanden, welche die Wasserlöslichkeit der Silicone bedingen. Erfindungsgemäß wird unter Wasserlöslichkeit verstanden, dass sich wenigstens 2 Gew.-% des mit hydrophilen Gruppen modifizierten Silicons in Wasser bei 20 °C lösen. Entsprechende hydrophile Substituenten sind beispielsweise Hydroxy-, Polyethylenglycol- oder Polyethylenglycol/Polypropylenglycol-Seitenketten sowie ethoxylierte Ester-Seitenketten. Erfindungsgemäß bevorzugt geeignet sind hydrophil modifizierte Silicon-Copolyole, insbesondere Dimethicone-Copolyole, die beispielsweise von Wacker-Chemie unter der Bezeichnung Belsil® DMC 6031, Belsil® DMC 6032, Belsil® DMC 6038 oder Belsil® DMC 3071 VP bzw. von Dow Corning unter der Bezeichnung DC 2501 im Handel sind. Besonders bevorzugt geeignet ist die Verwendung von Belsil® DMC 6038, da es die Formulierung hochtransparenter Zusammensetzungen ermöglicht, die beim Verbraucher eine höhere Akzeptanz erreichen. Erfindungsgemäß kann auch ein beliebiges Gemisch dieser Silicone eingesetzt werden.
Die Menge des hydrophil modifizierten Silicons oder des Alkohol-Gemisches in den erfindungsgemäßen Zusammensetzungen beträgt 0,5 - 10 Gew.-%, bevorzugt 1 - 8 Gew.-% und besonders bevorzugt 2 - 6 Gew.-%, bezogen auf das Gesamtgewicht der Zusammensetzung.

Die erfindungsgemäßen Zusammensetzungen können weiterhin wenigstens ein wasserlösliches Tensid enthalten. Als wasserlösliche Tenside eignen sich grundsätzlich alle in dem System zu 1 Gew.-% bei 20°C löslichen und in Wasser bei 20°C zu mindestens 1 Gew.-% löslichen Tenside. Obwohl die Struktur und lonogenität an sich unerheblich sind, scheinen nichtionische Tenside, insbesondere die bei Normaltemperatur (20°C) festen Anlagerungsprodukte des Ethylenoxids an Fettstoffmoleküle mit wenigstens einer alkoxylierbaren Gruppe, bevorzugt geeignet zu sein. Solche geeigneten Tenside sind z.B. die Anlagerungsprodukte von 10 - 40 Mol Ethylenoxid an lineare Fettalkohole mit 16 - 22 C-Atomen, an Fettsäuren mit 12 - 22 C-Atomen, an Fettsäurealkanolamide, an Fettsäuremonoglyceride, an Sorbitan-Fettsäuremonoester, an Fettsäurealkanolamide, an Fettsäureglyceride, z.B. an gehärtetes Rizinusöl, an Methylglucosidmonofettsäureester und Gemische davon.

Die erfindungsgemäßen Zusammensetzungen enthalten in einer bevorzugten Ausführungsform mindestens einen Antitranspirant-Wirkstoff. Als Antitranspirant-Wirkstoffe eignen sich wasserlösliche adstringierende metallische Salze, insbesondere anorganische und organische Salze des Aluminiums, Zirkoniums und Zinks bzw. beliebige Mischungen dieser Salze. Erfindungsgemäß wird unter Wasserlöslichkeit eine Löslichkeit von wenigstens 5 g Aktivsubstanz pro 100 g Lösung bei 20 °C verstanden. Erfindungsgemäß verwendbar sind beispielsweise Alaun (KAl(SO₄)₂ · 12 H₂O), Aluminiumsulfat, Aluminiumlactat, Natrium-Aluminium-Chlorhydroxylactat, Aluminiumchlorhydroxyallantoinat, Aluminiumchlorohydrat, Aluminiumsulfocarbolat, Aluminium-Zirkonium-Chlorohydrat, Zinkchlorid, Zinksulfocarbolat, Zinksulfat, Zirkoniumchlorohydrat und Aluminium-Zirkonium-Chlorohydrat-Glycin-Komplexe. Bevorzugt enthalten die Zusammensetzungen ein adstringierendes Aluminiumsalz, insbesondere Aluminiumchlorohydrat, und/oder eine Aluminium-Zirkonium-Verbindung. Die Antitranspirant-Wirkstoffe werden bei wässrigen Applikationen als wässrige Lösungen eingesetzt. In wasserfreien Zusammensetzungen werden die Antitranspirant-Wirkstoffe in fester Form eingesetzt. Sie sind in den erfindungsgemäßen Zusammensetzungen in einer Menge von 1 - 40 Gew.-%, vorzugsweise 5 - 30 Gew.-% und insbesondere 10 - 25 Gew.-% enthalten (bezogen auf die Menge der Aktivsubstanz in der Gesamtzusammensetzung). Aluminiumchlorohydrate werden beispielsweise pulverförmig als Micro Dry® Ultrafine von Reheis, als Chlorhydrol®, in aktivierter Form als Reach® 501 von Reheis sowie in Form einer wäßrigen Lösung als Locron® L von Clariant vertrieben. Unter der Bezeichnung Reach® 301 wird ein Aluminiumsesquichlorohydrat von Reheis angeboten. Auch die Verwendung von Aluminium-Zirkonium-Tetrachlorohydrex-Glycin-Komplexen, die beispielsweise von Reheis unter der Bezeichnung Rezal® 36G im Handel sind, ist erfindungsgemäß besonders vorteilhaft.

Weiterhin können die erfindungsgemäßen Zusammensetzungen keimhemmende oder desodorierende Wirkstoffe enthalten. Geeignet sind insbesondere Organohalogenverbindungen sowie -halogenide, quartäre Ammoniumverbindungen, eine Reihe von Pflanzenextrakten und Zinkverbindungen. Bevorzugt sind Chlorhexidin und Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar.

Weitere antibakteriell wirksame Deodorans-Wirkstoffe sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide.
Flüssige und gelförmige Darreichungsformen können Verdickungsmittel enthalten, z. B. Celluloseether, wie Hydroxypropylcellulose, Hydroxyethylcellulose und Methylhydroxypropylcellulose, verdickende Polymere auf Basis von Polyacrylaten, die gewünschtenfalls vernetzt sein können, z. B. die Carbopoltypen oder Pemulen® -Produkte, oder auf Basis von Polyacrylamiden oder sulfonsäuregruppenhaltigen Polyacrylaten, z. B Sepigel® 305 oder Simulgel® EG, weiterhin anorganische Verdicker, z. B. Bentonite und Hectorite (Laponite®).
Die erfindungsgemäßen Zusammensetzungen können weitere kosmetisch und dermatologisch wirksame Stoffe enthalten, wie beispielsweise entzündungshemmende Substanzen, Feststoffe, ausgewählt aus Kieselsäuren, z. B. Aerosil®-Typen, Kieselgelen, Siliciumdioxid, Tonen, z. B. Bentonite oder Kaolin, Magnesiumaluminiumsilikaten, z. B. Talkum, Bornitrid, Titandioxid, das gewünschtenfalls beschichtet sein kann, gegebenenfalls modifizierten Stärken und Stärkederivaten, Cellulosepulvern und Polymerpulvern, desweiteren Pflanzenextrakte, Proteinhydrolysate, Vitamine, Parfümöle, Sebostatika, Anti-Akne-Wirkstoffe sowie Keratolytika.

Die kosmetischen Deodorant- oder Antitranspirant-Zusammensetzungen, die die erfindungsgemäß verwendeten 5-Lipoxigenase-Inhibitoren enthalten, können, soweit sie flüssig vorliegen, auf flexible und saugfähige Träger aufgebracht und als Deodorant- oder Antitranspirant-Tücher oder Schwämmchen angeboten werden. Als flexible und saugfähige Träger im Sinne der Erfindung eignen sich z. B. Träger aus Textilfasern, Kollagen oder polymeren Schaumstoffen. Als Textilfasern können sowohl Naturfasern wie Cellulose (Baumwolle, Leinen), Seide, Wolle, Regeneratcellulose (Viskose, Rayon), Cellulosederivate als auch synthetische Fasern wie z.B. Polyester, Polyacrylnitril, Polyamid- oder Polyolefinfasern oder Mischungen solcher Fasern gewebt oder ungewebt verwendet werden. Diese Fasern können zu saugfähigen Wattepads, Vliesstoffen oder zu Geweben oder Gewirken verarbeitet sein. Auch flexible und saugfähige polymere Schaumstoffe, z. B. Polyurethanschäume und Polyamidschäume sind geeignete Substrate. Das Substrat kann eine, zwei, drei sowie mehr als drei Lagen aufweisen, wobei die einzelnen Lagen aus gleichen oder unterschiedlichen Materialien bestehen können. Jede Substratschicht kann eine homogene oder eine inhomogene Struktur mit beispielsweise verschiedenen Zonen unterschiedlicher Dichte aufweisen.
Als saugfähig im Sinne der Erfindung sind solche Trägersubstrate anzusehen, die bei 20° C wenigstens 10 Gew.-%, bezogen auf das Trockengewicht, an Wasser adsorptiv bzw. kapillar binden können. Bevorzugt eignen sich aber solche Träger, die wenigstens 100 Gew.-% Wasser adsorptiv und kapillar binden können.
Die Ausrüstung der Trägersubstrate erfolgt in der Weise, daß man die saugfähigen, flexiblen Trägersubstrate, bevorzugt aus Textilfasern, Kollagen oder polymeren Schaumstoffen mit den erfindungsgemäßen Zusammensetzungen behandelt bzw. ausrüstet und gegebenenfalls trocknet. Dabei kann die Behandlung (Ausrüstung) der Trägersubstrate nach beliebigen Verfahren, z. B. durch Aufsprühen, Tauchen und Abquetschen, Durchtränken oder einfach durch Einspritzen der erfindungsgemäßen Zusammensetzung in die Trägersubstrate erfolgen.

Erfindungsgemäß bevorzugt ist weiterhin die Darreichungsform als Aerosol, wobei die kosmetische Zusammensetzung ein Treibmittel, ausgewählt aus Propan, Butan, Isobutan, Pentan, Isopentan, Dimethylether, Fluorkohlenwasserstoffen und Fluorchlorkohlenwasserstoffen sowie Mischungen hiervon enthält.

Die folgenden Beispiele sollen die Erfindung verdeutlichen, ohne sie hierauf zu beschränken.

### Beispielrezepturen

| **Wasserfreie tensidhaltige AT-Stifte (Angaben in Gewichtsteilen)** | | | | | |
|---|---|---|---|---|---|
| | **1.1** | **1.2** | **1.3** | **1.4** | **1.5** |
| Eutanol® G 16 | 10 | - | - | 15 | 10 |
| Cetiol® OE | - | 10 | 15 | - | - |
| Ucon Fluid® AP | 5 | 5 | 5 | 5 | 5 |
| Cutina® HR | 6 | 6 | 6 | 6 | 6 |
| Lorol® C 18 | 20 | 20 | 20 | - | 20 |
| Lanette® O | - | - | - | 20 | - |
| Eumulgin® B 3 | 3 | 3 | 3 | 3 | 3 |
| Cutina® E 24 PF | - | - | - | - | 5 |
| Aluminiumchlorohydrat | 20 | 20 | 20 | 20 | 20 |
| Talkum | 8 | 8 | 8 | 8 | 8 |
| Seppicalm | 0,5 | - | - | - | - |
| Drieline | - | 0,5 | - | - | - |
| α-Liponsäure | - | - | 0,1 | - | - |
| Phytocohesine | - | - | - | 0,1 | - |
| α-Bisabolol | - | - | - | - | 0, 5 |
| Siliconöl DC® 245 | ad 100 | ad 100 | ad 100 | ad 100 | ad 100 |

| **Sprühfähige, translucente Antitranspirant-Mikroemulsionen (Angaben in Gew.-%)** | | | | | |
|---|---|---|---|---|---|
| | **2.1** | **2.2** | **2.3** | **2.4** | **2.5** |
| Plantaren® 1200 | 1,71 | 1,71 | - | 1,71 | 1,71 |
| Plantaren® 2000 | 1,14 | 1,39 | 2,40 | 1,14 | 1,39 |
| Glycerinmonooleat | 0,71 | 0,71 | - | 0,71 | 0,71 |
| Dioctylether | 4,00 | 4,00 | 0,09 | 4,00 | 4,00 |
| Öctyldodecanol | 1,00 | 1,00 | 0,02 | 1,00 | 1,00 |
| Parfümöl | 1,00 | 1,00 | 1,00 | 1,00 | 1,00 |
| Aluminiumchlorohydrat | 8,00 | 5,00 | 5,00 | - | - |
| 1,2-Propylenglycol | 5,00 | 5,00 | - | 5,00 | 5,00 |
| Glycerin | - | - | 5,00 | - | - |
| Seppicalm | 1,0 | - | - | - | - |
| Drieline | - | 0,6 | - | - | - |
| Phytocohesine | - | - | 0,5 | - | - |
| α-Bisabolol | - | - | - | 0,5 | - |
| α-Liponsäure | - | - | - | - | 0,1 |
| Wasser | ad 100 | ad 100 | ad 100 | ad 100 | ad 100 |

| **Antitranspirant-Spray vom Suspensionstyp (Angaben in Gew.-%)** | | | |
|---|---|---|---|
| | **3.1** | **3.2** | **3.3** |
| DC-245 | 10,0 | 10,0 | 10,0 |
| Isopropylmyristat | 5,0 | 5,0 | 5,0 |
| Aluminiumchlorohydrat-Pulver | 5,0 | 5,0 | 5,0 |
| Aerosil® R 972 | 2,0 | 2,0 | 2,0 |
| Seppicalm | 0,5 | - | - |
| Drieline | - | 0,2 | - |
| Phytocohesine | - | - | 0,2 |
| n-Butan | ad 100 | ad 100 | ad 100 |

### Antitranspirant-Tücher

Für die erfindungsgemäße Ausführungsform als Antitranspirant-Tuch wurde ein einlagiges Substrat aus 100 % Viskose mit einem Flächengewicht von 50 g/m² mit jeweils 75 g der Beispielemulsionen 2.1 bzw. 2.2 bzw. 2.3 pro Quadratmeter beaufschlagt, in Tücher geeigneter Größe geschnitten und in Sachets verpackt.

| **Rohstoff** | **Beschreibung/INCI** | **Herstellerfirma** |
|---|---|---|
| Aethoxal® B | PPG-5-Laureth-5 | Cognis |
| Cetiol® OE | Dicaprylyl Ether | Cognis |
| Cremophor® RH 455 | PEG-40 Hydrogenated Castor Oil, Aktivsubstanz 90 % | BASF |
| Cutina E 24 | PEG-20 Glyceryl Stearate | Cognis |
| Cutina® FS 45 | Palmitic Acid, Stearic Acid, Aktivsubstanz mind. 92 % | Cognis |
| Cutina® HR | Hydrogenated Castor Oil | Cognis |
| DC® 245 | Cyclopentasiloxane | Dow Corning |
| Drieline | Sorbitol, Yeast Extract | Lanatech |
| Eumulgin® B 3 | Ceteareth-30 | Cognis |
| Eutanol® G 16 | Hexyldecyl Stearate | Cognis |
| Lanette® O | Cetearyl Alcohol | Cognis |
| Lorol® C 18 | Stearyl Alcohol | Cognis |
| Mergital® CS 11 | Ceteareth-11 | Cognis |
| Phytocohesine | Sodium Beta-Sitosterylsulfate | Vincience |
| Plantaren® 1200 | Lauryl Glucoside, ca. 50 % Aktivsubstanz | Cognis |
| Plantaren® 2000 | Decyl Glucoside, ca. 50 % Aktivsubstanz | Cognis |
| Seppicalm | Sodium Cocoyl Aminoacids, Sarcosine, Potassium Aspartate, Magnesium Aspartate | Seppic |
| Ucon Fluid® AP | PPG-14 Butyl Ether | Amerchol (Union' Carbide) |

### Überprüfung der inhibitorischen Wirkung der 5-Lipoxigenase-Inhibitoren (in vitro)

Das Enzym 5-Lipoxigenase katalysiert die Umsetzung von Arachidonsäure zu den Leukotrienen LTB₄, LTC₄, LTD₄ und LTE₄. Leukotriene sind Mediatoren in entzündlichen und allergischen Reaktionen von Granulozyten, Mastzellen, Monozyten und Macrophagen. Auch in den Keratinozyten der Haut werden bei entzündlichen Reaktionen Leukotriene gebildet. Inhibitoren der Leukotrien-Synthese können demnach eine entzündungshemmende bzw. hautberuhigende Wirkung haben.

### Bereitstellung der 5-Lipoxigenase

Die 5-Lipoxigenase wurde aus menschlichen Leukämie-Zelllinien durch Zugabe von Dimethylsulfoxid (DMSO), TGF-β1 (transforming growth factor β1) und Dihydroxyvitamin D3 differenziert und dabei zugleich die 5-Lipoxigenase induziert. Nach 4 Tagen im Differenzierungsmedium wurden die Zellen geerntet, in einem Glucose-haltigen PBS-Puffer aufgenommen und durch Ultraschallbehandlung aufgeschlossen. Größere Zellbestandteile wurden bei 100 000 g abzentrifugiert, wobei 5-Lipoxigenase im Überstand verblieb.

### Messung der 5-Lipoxigenase-Inhibierung

Die Bestimmung der 5-Lipoxigenase-Inhibitionswirkung erfolgte gemäß Werz et al., Naunyn-Schmiedeberg's Arch. Pharmacol., 1997, Vol. 456, 441 - 445.

Dem Überstand aus der 5-Lipoxigenase-Herstellung wurden Adenosintriphosphat (ATP) und die zu testenden Rohstoffe zugesetzt. Im vorliegenden Falle wurden 100 µg Rohstoff, so wie er ist, pro Milliliter Überstand eingesetzt. Als Lösemittel wurden je nach Substanz Wasser, Ethanol, DMSO oder Chloroform verwendet.

Die Ansätze wurden für 30 Sekunden bei 37° C vorinkubiert. Anschließend wurde die 5-Lipoxigenase-Reaktion durch Zusatz von Arachidonsäure gestartet. Nach 10 Minuten Reaktionszeit wurde die Reaktion durch Zugabe von Methanol gestoppt. Die durch die Einwirkung der 5-Lipoxigenase auf Arachidonsäure produzierten Leukotriene wurden mittels HPLC quantifiziert.
Die Aktivität von 5-Lipoxigenase ohne Zugabe von Testsubstanz wurde zu 100 % gesetzt und die Aktivität der 5-Lipoxigenase mit Hemmstoff darauf bezogen. In Tabelle 1 ist die Hemmwirkung der einzelnen Testsubstanzen in % angegeben.

**Tabelle 1:**

| **Verschiedene Wirkstoffe als 5-Lipoxigenase-Inhibitor** | | |
|---|---|---|
| **Rohstoff** | **Hemmwirkung** | **Lösemittel** |
| Seppicalm | 72,0 % | Ethanol |
| Drieline | 40,2 % | Wasser |
| α-Bisabolol | 90,3 % | Ethanol |
| α-Liponsäure | 40,4% | Ethanol |
| Phytocohesine | 62,8 % | DMSO |

## Patentansprüche

1. Verwendung von mindestens einer 5-Lipoxigenase-inhibierenden Substanz, ausgewählt aus
Aminodicarbonsäuren mit einer C-Kettenlänge von 3 - 6 Kohlenstoffatomen sowie deren physiologisch verträglichen Salzen,
N-alkylierten C₂-C₁₁-Aminosäuren mit C₁-C₂₂-Alkylresten sowie deren physiologisch verträglichen Salzen,
N-acylierten C₂-C₁₁-Aminosäuren mit C₂-C₂₂-Acylresten sowie deren physiologisch verträglichen Salzen,
Hefeextrakten,
α-Bisabolol,
α-Liponsäure und
den physiologisch verträglichen Salzen von Sterolsulfaten,
in einer kosmetischen Deodorant- oder Antitranspirant-Zusammensetzung zur Verringerung des durch die Bildung von gesättigten und ungesättigten C₆-C₁₂-Aldehyden aus den ungesättigten Fettsäuren der Haut und der Kopfhaut verursachten Körpergeruchs.

2. Verwendung gemäß Anspruch 1, **dadurch gekennzeichnet, dass** die Aminodicarbonsäuren mit einer C-Kettenlänge von 3 - 6 Kohlenstoffatomen sowie deren physiologisch verträglichen Salzen ausgewählt sind aus Asparaginsäure und ihren physiologisch verträglichen Salzen.

3. Verwendung gemäß einem der vorstehenden Ansprüche, **dadurch gekennzeichnet, dass** die N-alkylierten C₂-C₁₁-Aminosäuren mit C₁-C₂₂-Alkylresten sowie deren physiologisch verträglichen Salze ausgewählt sind aus N-Methylglycin.

4. Verwendung gemäß einem der vorstehenden Ansprüche, **dadurch gekennzeichnet, dass** die N-acylierten C₂-C₁₁-Aminosäuren mit C₂-C₂₂-Acylresten sowie deren physiologisch verträglichen Salze ausgewählt sind aus Natriumcocoylaminosäuren, Natriumoctanoylglutamat, Natriumdecanoylglutamat, Natriumlauroylglutamat, Natriummyristoylglutamat, Natriumcetoylglutamat und Natriumstearoylglutamat und den Lauroyl-Derivaten von aus Getreidepflanzen gewonnenen Aminosäuren.

5. Verwendung gemäß einem der vorstehenden Ansprüche, **dadurch gekennzeichnet, dass** die physiologisch verträglichen Salze von Sterolsulfaten ausgewählt sind aus den Salzen von β-Sitosterolsulfat.

6. Verfahren zur Verringerung von Körpergeruch mittels Inhibierung von 5-Lipoxigenase auf der Haut, **dadurch gekennzeichnet, dass** eine kosmetische Deodorant- oder Antitranspirant-Zusammensetzung, enthaltend mindestens eine 5-Lipoxigenase-inhibierende Substanz, ausgewählt aus
Aminodicarbonsäuren mit einer C-Kettenlänge von 3 - 6 Kohlenstoffatomen sowie deren physiologisch verträglichen Salzen,
N-alkylierten C₂-C₁₁-Aminosäuren mit C₁-C₂₂-Alkylresten sowie deren physiologisch verträglichen Salzen,
N-acylierten C₂-C₁₁-Aminosäuren mit C₂-C₂₂-Acylresten sowie deren physiologisch verträglichen Salzen,
Hefeextrakten,
α-Bisabolol,
α-Liponsäure und
den physiologisch verträglichen Salzen von Sterolsulfaten,
auf die Haut, insbesondere auf die Haut der Achselhöhlen, aufgetragen wird.

7. Verwendung von α-Liponsäure gegen Hautirritationen, zur Entzündungshemmung und zur Hautberuhigung.
